# EUROPEAN PATENT APPLICATION

(11) **EP 1 873 318 A1**
(43) Date of publication of application: **02.01.2008**
(21) Application number: 06722310.7
(22) Date of filing: 11.04.2006
(51) Int. Cl.: E03D 9/04, A47K 11/02

(54) **HUMAN WASTE AUTOMATIC PROCESSOR**

(30) Priority: 11.04.2005 CN 200510034007; 18.04.2005 CN 200510034171
(71) Applicant: Liu, Zhenhua, Changsha City Hunan 410016 (CN)
(72) Inventor: Liu, Zhenhua, Changsha City Hunan 410016 (CN)
(74) Representative: Rambelli, Paolo
(86) International application number: PCT/CN2006/000658
(87) International publication number: WO 2006/108356

(57) **Abstract**

The present aspect relates to a convenient disposal machine for human excrement. The disposal machine for human excrement includes: a excretion pool, a sewage bucket and a controller. The bottom of the excretion pool is connected with a blow-off pipe, and the other end of the blow-off pipe is connected with the opening of the sewage bucket, the top of the sewage bucket is connected with the air inlet of the compressor via a fitting pipe, and the air outlet of the compressor is connected with an air cleaner.

## Description

### BACKGROUND OF THE INVENTION

### 1. FIELD OF THE INVENTION

The present invention relates to a cleaning and caring machine, i.e., a disposal machine for human excrement, and more particularly to a disposal machine for human excrement used to dispose the feces and urine of the people who are unable to move easily. It also relates to a method for operating the disposal machine for human excrement.

### 2. DISCUSSION OF THE RELATED ART

Chinese Patent Publication CN2520165Y published on November, 13th, 2002 discloses "A sanitation device used with sickbed" (also known as a disposal machine for human excrement), which is provided on the back of the sickbed, including a flusher, a excrement container, a controller which is connected with the flusher through electrical wires, and a bracket fixed on the back of the sickbed. The flusher and the excrement container are arranged crosswise at the side frame and the central area of the bracket, respectively, with the flusher located above the excrement container. However, the existing sanitation device for use in a sickbed only introduces an excrement container and a flusher to the sickbed, and it must be used together the sickbed, which is inconvenient to use. Besides, no deodorization treatment is performed on the dejecta.

### SUMMARY OF THE INVENTION

An object of the present invention is to provide a convenient disposal machine for human excrement which overcomes the inconvenience with the existing disposal machine for human excrement.

The object of the present invention is achieved by a disposal machine for human excrement, which includes a excretion pool situated within a seat pad, a sewage bucket and a controller, wherein a blow-off pipe is such configured that it has one end connected to the bottom of the excretion pool within the seat pad and the other end connected to the sewage bucket, and the top of the sewage bucket is connected with a compressor via a fitting pipe, the compressor connected to an air cleaner.. When the processor is in use, the compressor operates and generates negative pressure in the sewage bucket, so the dejecta in the excretion pool within the seat pad is driven by the pressure to flow into the sewage bucket via the blow-off pipe. The odor in the sewage bucket is purified by the air cleaner before it is sent back to the seat pad by the compressor. Of course, as is known to those skilled in the art, a vacuum pump and other pumping device may be used instead of the compressor used herein.

According to another aspect of the present invention, the blow-off pipe is connected to the top surface of the sewage bucket for preventing the dejecta in the sewage bucket from blocking the outlet of the blow-off pipe, thus ensuring of the pumping effect of the compressor upon the sewage bucket.

According to another aspect of present invention, a water pipe is such configured that it has one end connected to the upper part of the excretion pool and the other end to an outlet of a three-way valve. The inlet of the three-way valve is connected to the outlet of an electromagnetic pump, the inlet of which is connected with the sink.. After the user excretes, the controller controls the electromagnetic pump to rotate so that the water sucked by the electromagnetic pump from the sink is ejected via the water pipe to wash the wastes as well as the private parts of the user. Herein, the upper part of the excretion pool connected with the water and air pipe is described with respect to the joint between the above mentioned blow-off pipe and the excretion pool.

According to another aspect of the present invention, a heater (also called as a heating tube) is provided in the sink. Generally, the heater is an electrical heat tube, and of course some other heating devices as known to those skilled in the art.

According to another aspect of the present invention, a water and air pipe is such configured that it has one end connected to the upper part of the excretion pool and the other end to a vacuum pump via a one-way check valve 987. In use, the controller controls the vacuum pump to discharge air, which then blows to the wastes as well as the private parts of the user. The working cycle of the vacuum pump is carried out intermittently according to the working procedure of MCU. Herein, the upper part of the excretion pool connected with the air pipe is described with respect to the joint between the above mentioned blow-off pipe and excretion pool. Of course, as also known to those skilled in the art, a blower, a compressor and some other air supply device can also be used instead of the vacuum pump.

According to another aspect of the present invention, a negative ion generator is defined in the inlet passage or the outlet passage of the vacuum pump. When the vacuum pump is operating, the negative ions drawn in the pump can sterilize and disinfect the private parts of the user and the excretion pool.

According to another aspect of the present invention, a heater is defined in the inlet passage or the outlet passage of the vacuum pump for heating the air directed into the excretion pool.

According to another aspect of the present invention, the air pipe is partially immersed into the water in the sink where a heater is provided. The airflow inside the air pipe absorbs heat from the sink when passing through the part of the air pipe immersed into the sink. Therefore, there is no need to configure a heater for heating the air on the inlet passage or the outlet passage.

According to another aspect of the present invention, a conduit is configured in the processor in a way that it has one end connected to the upper part of the excretion pool and the other end to the outlet of a three-way valve, the inlets of the three-way valve connected with the water outlet of the electromagnetic pump. Another outlet of the three-way valve is connected with the outlet of the one-way check valve; and the water inlet of the electromagnetic pump is connected with the sink. The outlet of the vacuum pump is connected with the inlet of the one-way check valve; the water outlet of the electromagnetic pump is connected with the normal closed valve of the three-way valve. With the aid of the one-way check valve, the pathway of the vacuum pump keeps open during use. Here, the upper part of the excretion pool connected with the water and air pipe is described with respect to the joint between the above-mentioned blow-off pipe and excretion pool.

According to yet another aspect of the present invention, a heater is provided in the sink; and part of the air pipe is immersed in the water in the sink.

According to an improved embodiment of the present invention, a disposal machine for human excrement is provided, comprising: a seat pad, a sewage disposal assembly to collect excrement from the seat pad, a water supply assembly to feed water into the seat pad, an air supply assembly to feed air into the seat pad, and a control assembly to control operations of the sewage disposal assembly, water supply assembly and air supply assembly.

According to a further improvement of the present invention, the sewage disposal assembly includes a sewage bucket, an air cleaner and a compressor; the water supply assembly includes a water tank, a sink located under the water tank and an electromagnetic pump connected with the sink; and the air supply assembly includes a vacuum pump and a heating component.

According to one feature of the present invention, in a standby state, the vacuum pump drives the air in the seat pad to flow through the sewage bucket, the air cleaner, and then through the heating component where the air gets heated, and finally flow back into the seat pad in order to maintain a warm and ventilated atmosphere in the seat pad; and in a working state, the compressor drives the air in the seat pad to flow through the sewage bucket, the air cleaner, and finally back into the seat pad in order to main a pressure balance in the seat pad. The water supply assembly further includes a heater defined in the sink for heating water that, once heated, is driven by the electromagnetic pump to flow into the seat pad via the water pipe and the passages within the seat pad in the working state.

In addition, the present invention also provides a tournure for the disposal machine for human excrement, which has an appropriate opening for accommodating the seat pad. The tournure makes the user feel more comfortable.

Furthermore, the present invention also provides a method for operating the disposal machine for human excrement, which includes a working state when activated, a sleeping state when shut down, and a standby state after a task is finished.

According to the method of operating the disposal machine for human excrement, in the working state, a suction device operates to generate a negative pressure in the seat pad and drives air into the seat pad, when the electromagnetic pump operates to feed water into the seat pad simultaneously; and in the standby state, an air supply device operates to supply warm wind into the seat pad.

According to the technical solutions employed in the present invention, by means of the disposal machine for human excrement of the present invention, the human waste is pumped away while being defecated. Therefore, the excretion pool can be made into a thin shape having a tournure so that it can be put under the private parts of the bedridden patient directly. That is, the tournure and the seat pad are combined together. Generally, the excretion pool does not have to be taken out, so it is applicable to different beds without having to modify the sickbed, and thus convenient for use. In addition, according to an improved technical solution, the water and the air sent to the seat pad are heated, and an air pressure balance can also be maintained in the seat pad, thus increasing the comfortability of the disposal machine for human excrement in accordance with the present invention. Furthermore, a corresponding temperature detector and liquid level detector can also be provided in the automatic processor to enhance the safety performance of the disposal machine for human excrement. The use of a wireless remote controller makes it more efficient and convenient to control the disposal machine for human excrement.

### BRIEF DESCRIPTION OF THE DRAWINGS

The present invention will become more apparent after the following descriptions to the embodiments with reference to the figures, wherein, the figures are not drawn to true scale and some of the figures are enlarged and/or cut away for convenient illustration.

Fig.1 is a functional schematic view of the air way and the water way of the disposal machine for human excrement in accordance with one embodiment of the present invention.

Fig.2 is an exploded view for the disposal machine for human excrement in accordance with one embodiment of the present invention.

Fig.3 is a functional schematic view of the air way and the water way of the disposal machine for human excrement in accordance with another embodiment of the present invention.

Fig.4 is an exploded view of the disposal machine for human excrement in accordance with another embodiment of the present invention.

Fig.5 is a schematic view illustrating an overall profile of the disposal machine for human excrement in accordance with the present invention.

Fig.6A, 6B and 6C are schematic views illustrating the structure of the seat pad in accordance with the present invention from different view angles.

Fig.7 is a perspective view of the sewage bucket with a handle in accordance with the present invention, wherein, the sewage suction head seat is in open state.

Fig.8 is a plan view of the sewage bucket in accordance with the present invention, wherein, the sewage suction head seat and the sewage bucket lid are in open state.

Fig.9 is a partial cutaway view of the sewage bucket in accordance with the present invention, wherein, the sewage suction head seat and the sewage bucket lid are in closed state.

Fig.10 is a perspective view of the sewage bucket in accordance with the present invention, beside which is set a piece of clapboard with an overflow detector.

Fig.11 is a perspective view of the sewage bucket in accordance with the present invention, beside which is set a piece of clapboard with an overflow detector.

Fig.12 is a perspective view of the water tank in accordance with the present invention, wherein the water tank and the water tank lid are in disassembled state.

Fig. 13 is a perspective view of the water tank upside down in accordance with the present invention, wherein, the water tank lid is in open state.

Fig.14A to 14D are different views of the tournure in accordance with the present invention.

Fig.15A and 15B are different views of the combination of the tournure and the seat pad in accordance with the present invention.

Fig.16 is a block diagram of the control module of the disposal machine for human excrement in accordance with the present invention.

Fig.17 is a schematic view illustrating the circuit principle for the control module of the disposal machine for human excrement in accordance with the present invention.

Fig.18 is a schematic view illustrating the circuit principle for the remote controller of the disposal machine for human excrement in accordance with the present invention.

Fig. 19 is a flow chart illustrating the normal work flow 1 for the disposal machine for human excrement in accordance with the present invention.

Fig.20 is a flow chart illustrating the normal work flow 2 of the disposal machine for human excrement in accordance with the present invention.

Fig.21 is a work flow illustrating the alarming for the disposal machine for human excrement in accordance with the present invention.

Fig.22 is a schematic block diagram illustrating the timing (delaying) operation of the disposal machine for human excrement in accordance with the present invention.

List of partial elements in the figures

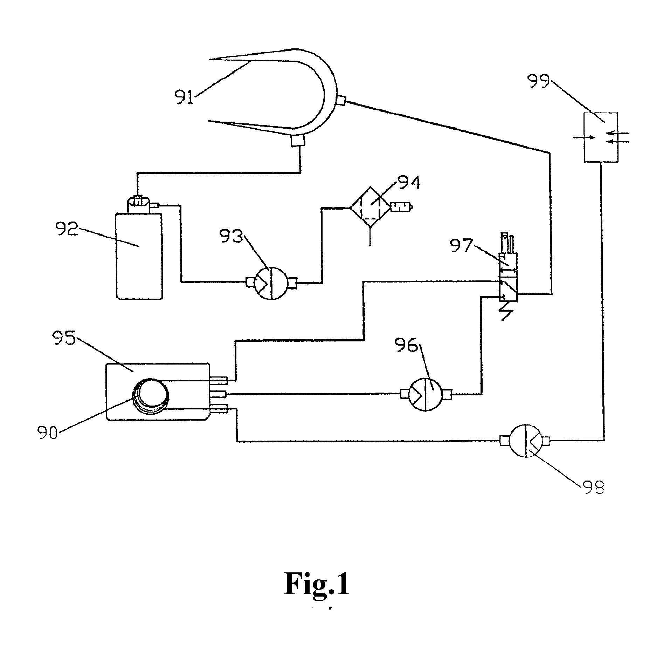

91. excretion pool, 92. sewage bucket, 93. compressor, 94. air cleaner, 95. sink, 90. heat conducting air duct, 96. electromagnetic pump, 97. three-way solenoid valve, 98. vacuum pump, 99. negative ion generator; (wherein, the above reference numbers are in fig. 1) 37. upper lid, 38. surface lid, 21. water tank, 22. water valve, 23. valve stem spring, 24. water tank sealing ring, 25. water tank lid, 26.water tank valve stem, 27. waterproof plate, 28. sink clapboard, 29. water-level detector, 30. heat conducting air duct, 31. sink, 32. heating tube, 33. air cleaner, 34. vacuum pump, 800. overflow detector, 35. Compressor, 36. electromagnetic pump, 11. Chassis, 12. winder, 13. winding Sensor, 14. base, 15. universal truckle, 41, 42. median clapboard, 441. mesh, 44. housing, 61. handle, 62. upper bracket, 63. connecting frame, 64. rod head, 45. suction head lid, 65. upper slide bar, 46. bent pipe, 66. upper steering knuckle, 67. lower steering knuckle, 47. sewage bucket sealed cap, 68. lower slide bar, 48. sewage bucket sealing ring, 49. valve, 69. fixing frame, 18. negative ion generator, 17. fan bracket, 16. fan, 71. connecting rod, 72. slide bar sleeve, 51. sewage bucket handle, 50. sewage bucket, 52. blow-off pipe, 55. three-way solenoid valve, 551. Outlet, 501. joint, 56. solenoid valve bracket, 100. sewage suction head seat, 101. air pipe joint, 102. buckle, 991. seat pad, 999. host machine, 992. water/air jet nozzle, 993. air jet nozzle, 994. water/air inlet nozzle, 995. discharge nozzle, 996.upper part, 997. lower part, 987. one-way valve, 989. passage, 941. overflow detector, 942. alarm, 966. tournure, 969. connecting wire, 968. zipper, 962. tournure opening.

### DESCRIPTION OF THE PREFERRED EMBODIMENTS

The words "above", "upper part", "under", "lower part", "left" and "right" as used in present specification are all relative, and should be adjusted accordingly when the specific view is changed, or the disposal machine and corresponding device operate in different situations. It should be pointed out that for the sake of illustration, similar elements in fig. 1 sometimes may be denoted by different reference numerals from the rest of the figures, but this does not mean that they are not similar elements or have different functions.

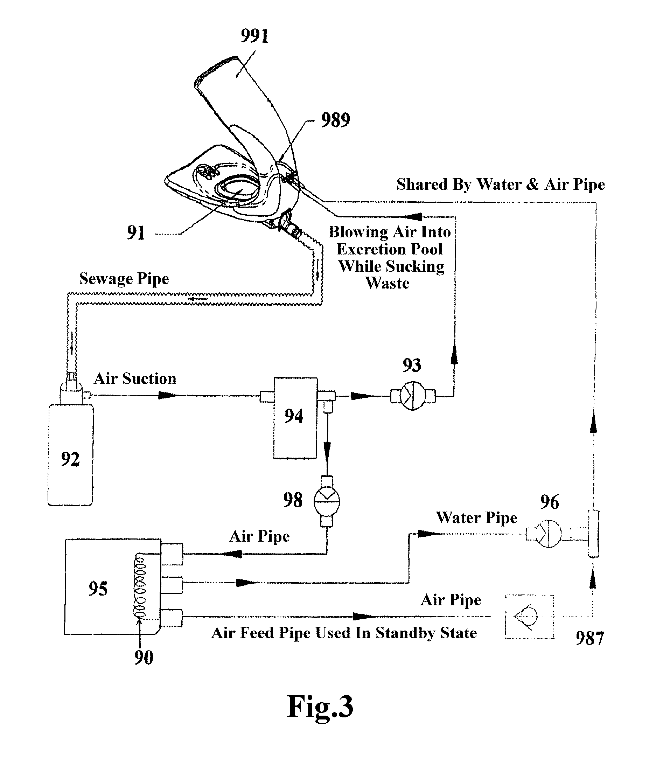

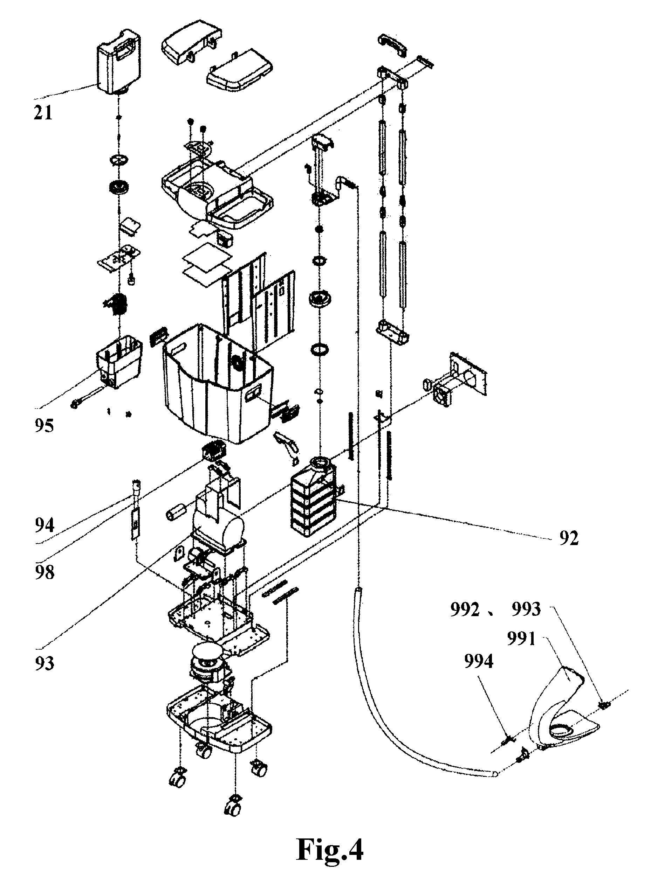

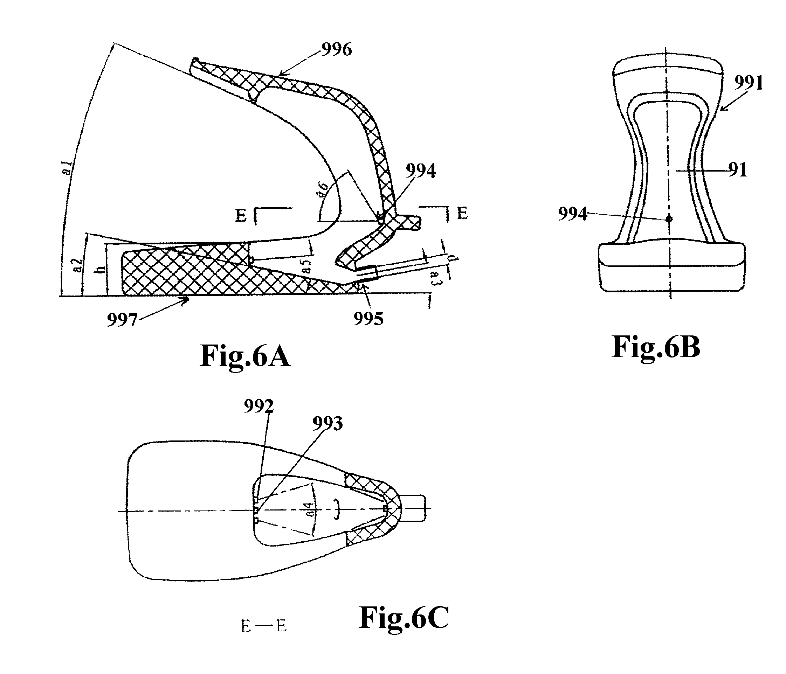

Referring to Fig.1, the cleaning and caring machine, i.e. the disposal machine for human excrement, is composed of a excretion pool 91, a sewage bucket 92, a compressor 93, an air cleaner 94, a sink 95(see fig.4), an electromagnetic pump 96, a three-way solenoid valve 97, a vacuum pump 98, a negative ion generator 99 (see Fig.1). The lines connecting each part represent the pipelines. The U-shaped or V-shaped opening of the excretion pool 91(see Fig.6B) faces left. The top of the sealed sewage bucket 92 is communicated with the bottom discharge nozzle of the excretion pool 91, and the air suction port of the compressor 93 (see Fig. 3) is connected at the top of the sewage bucket and is communicated with the internal cavity of the sewage bucket. The air suction port of the compressor 93 is connected with the air cleaner 94. The output port of the three-way solenoid valve 97 is connected to the upper part of the excretion pool 91 via a water and air pipe. The nozzle 994 of the conduit opens towards the excretion pool. The water outlet of the sink 95 with an electrical heat tube provided inside and filled with water is connected with the electromagnetic pump 96, the water outlet of which is connected with one inlet of the three-way solenoid valve 97. The air suction end of the vacuum pump 98 is connected with the negative ion generator 99; the air outlet end is connected with the inlet of the heat exchange air duct 90 that is immersed in the water in the sink; and the outlet of the heat exchange air duct 90 is connected with another inlet of the three-way solenoid valve 97. The heat exchange air duct 90 is part of the air outlet pipe of the vacuum pump 98. There is provided with a controller to control the switching between the compressor, the electromagnetic pump, the vacuum pump, the electrical heat tube and the three-way solenoid valve.

During use, the U-shaped or V-shaped excretion pool 91 is pushed leftward to situate between the legs of a lying patient, and is fixed to the hips of the body to form a seal to some extent. The compressor 93, the vacuum pump 98, the negative ion generator 99 and the electrical heat tube (of course, the electrical heat tube is controlled by a thermostat instead) are then switched on. After the patient excretes, under the control of the remote control, the vacuum pump sucks air with negative ions, and, having been subjected to a heat exchange between the heat exchange air duct 90 and the warm water in the sink, the warm air is directed to the feces and urine at the private parts of the patient via the three-way solenoid valve, and dries the skin of the patient. Thereafter, the three-way solenoid valve 97 and the electromagnetic pump 96 are turned on to drive the electromagnetic pump to spray water and wash the private parts of the patient. Upon washing, the electromagnetic pump and the three-way solenoid valve are turned off, when the vacuum pump 98 again supplies warm air to dry the private parts of the patient again. The controller may be configured as a remote controller to facilitate the patient to use it for his own.

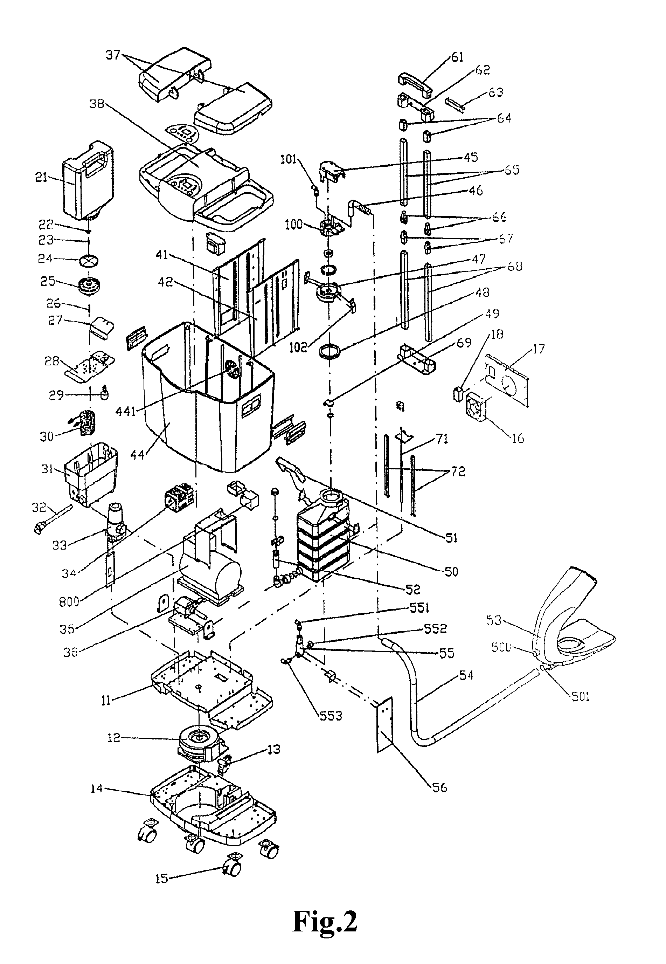

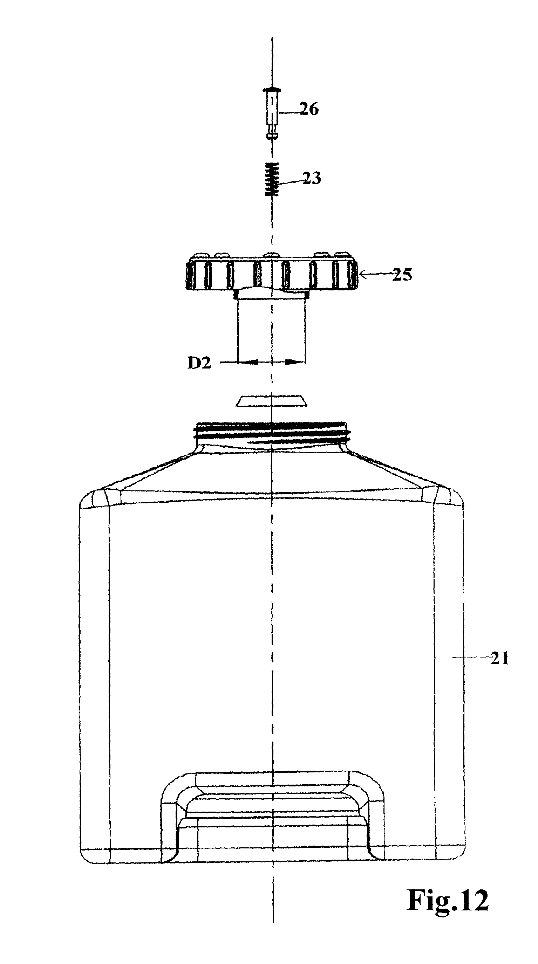

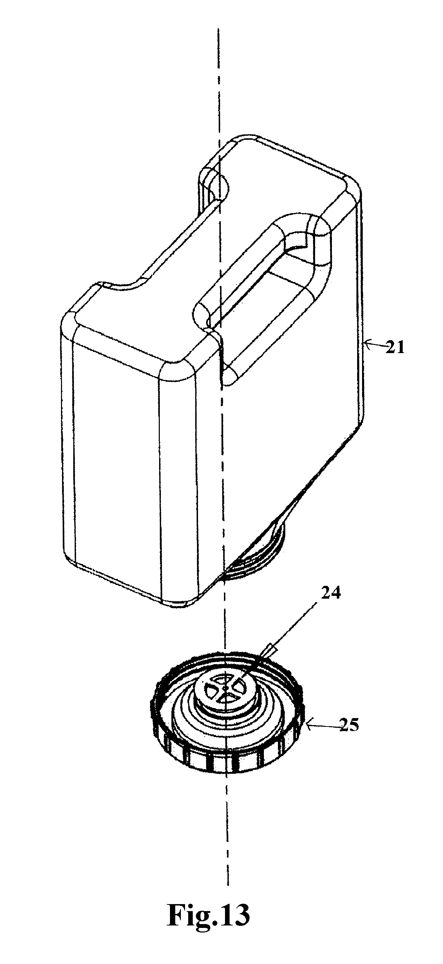

Referring to Fig.2, wherein some connecting lines are not shown, in the disposal machine for human excrement, the electrical heat tube 32 extends into the sink via a side port at the lower part of the sink 31, and the heat exchange air duct 30 is placed into the sink and immersed into water. Also referring to Fig.12 and Fig. 13, the water tank 21 filled with water is placed upside down into the sink, the opening of the water tank is sealed by a water valve 22, a valve stem spring 23, a water tank sealing ring 24 and a water tank lid 25 so that the water inside the tank cannot flow out before being put into the sink. There is provided a water level detector 29 (which controls the water level) in the sink 31 for controlling the water valve 22 in order to maintain the water level in the sink within a certain range. When the water in the water tank is used up, the water tank can be taken out and filled with water by opening the water tank lid. After the water tank is placed into the sink, the water discharge column in the sink 31 happens to open the valve stem 26 of the water tank lid, such that the water tank is operable to discharge water into the sink. The diameter D2 of the opening in the water tank lid 25 is designed to be about 1 mm to about 80 mm. When the water level in the sink is lower than a predetermined lower limit, the water tank will discharge water automatically. When the water level increases to a predetermined upper limit and higher than the water discharge port of the water tank, the water in the sink and the water tank is balanced with the outside atmospheric pressure, so the water tank will stop adding water into the sink immediately. Therefore, the present invention can guarantee that the water tank replenishes an appropriate amount of water into the sink automatically. When the water in the sink is insufficient as lower than the lower limit value set by the water level detector 29, a detection signal will be sent to a control module, which controls the device to stop working and effecting an alarming. The device will not resume operation until an appropriate amount of water is added (see Fig.2).

The water inlet of the electromagnetic pump 36 is communicated with the sink 31, while the water outlet is connected with the inlet 553 of the three-way solenoid valve 55. The sewage suction head seat 100 mounted with an air pipe 101 and a bent pipe 46 is fixed in the housing 44. Under the air suction head are a sewage bucket sealed cap 47 and a sewage bucket sealing ring 48. The air pipe joint 101 is connected with the air suction port of the air cleaner 33, while the air inlet of the compressor 35 is connected with the air outlet of the air cleaner 33. The bent pipe 46 is connected with the joint 501 at the bottom of the excretion pool 53 via a blow-off pipe 54(for example, a corrugated pipe). The sewage bucket 50 is placed into the housing 44 with the opening facing right towards the beneath of the suction head seat 100. The slide buckles 102 secure the slop nail 50 and open the valve 49(see Fig.2).

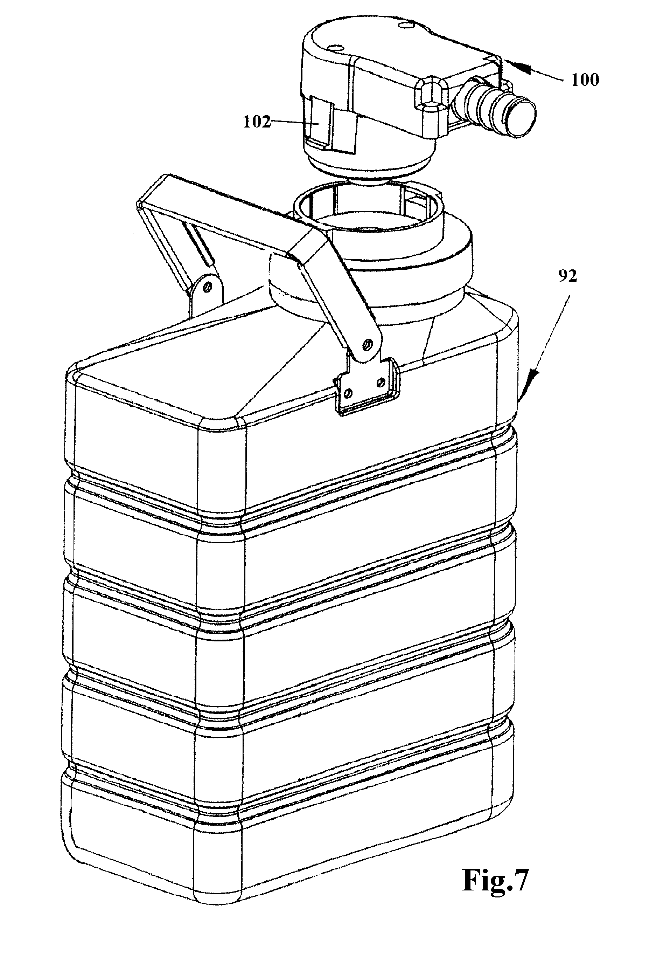

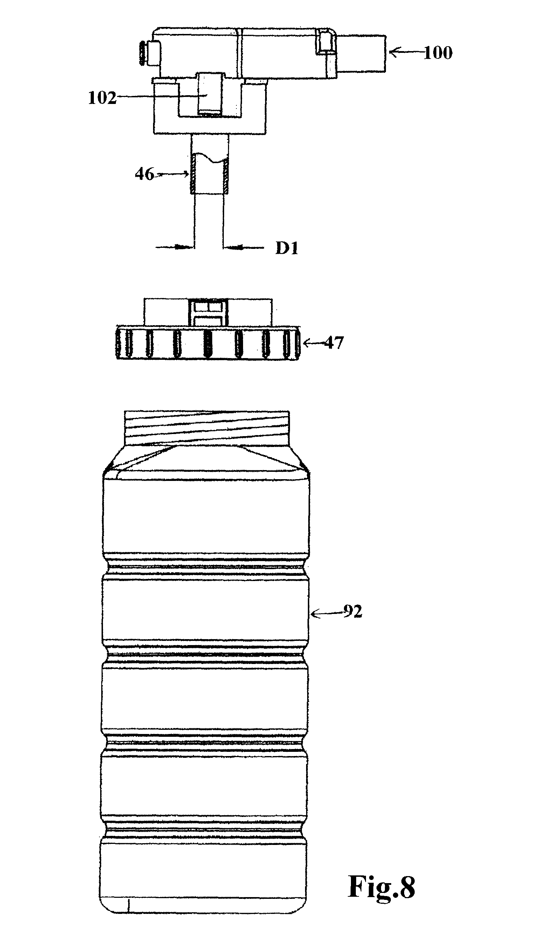

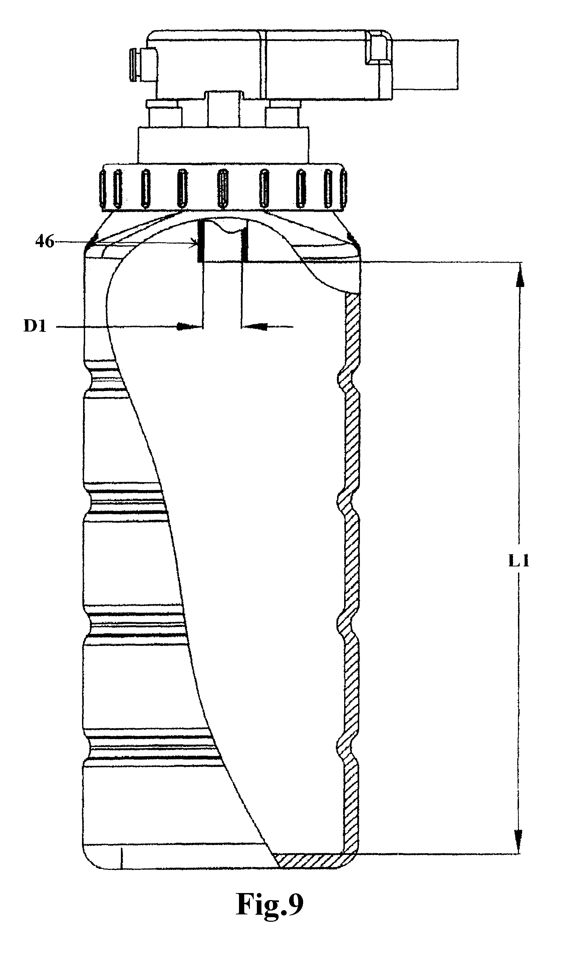

Also referring to Fig.7, two slide buckles 102 are designed on the sewage suction head seat 100. To connect the sewage suction head seat 100 with the sewage bucket, one only needs to tamp down the two slide buckles 102 with his index finger and thumb and press them down slightly. To disconnect them, it suffices to tamp down the two slide buckle 102 with the index finger and thumb and then bring them up slightly. As such, the sewage suction head seat 100 is easily operable. Also referring to Fig. 8 and Fig. 9,the bent pipe 46 inserted from the top of the sewage bucket has an diameter D1 ranging about 1 mm to about 50 mm, and is located at a distance L1 ranging about 1 cm to about 80 cm to the bottom of the sewage bucket.

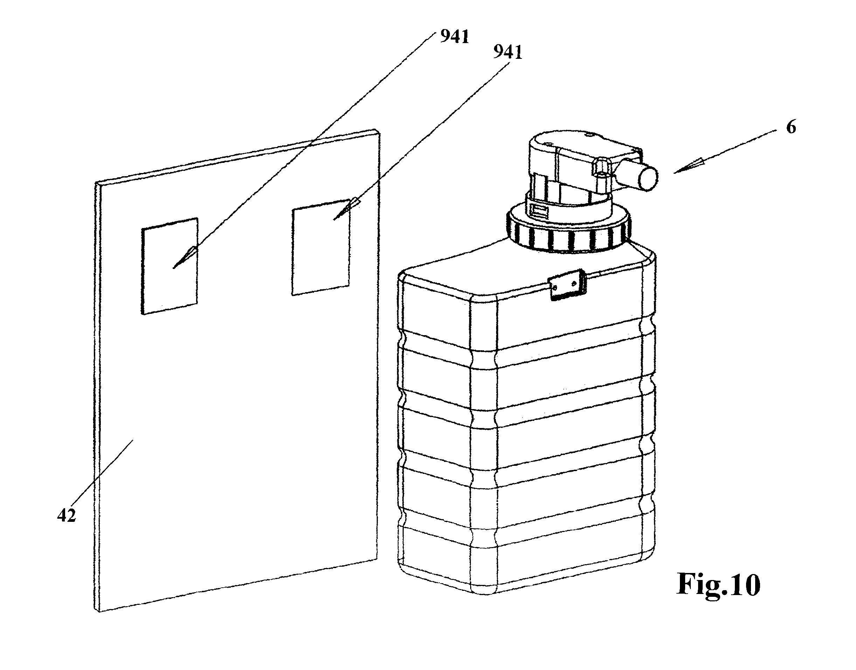

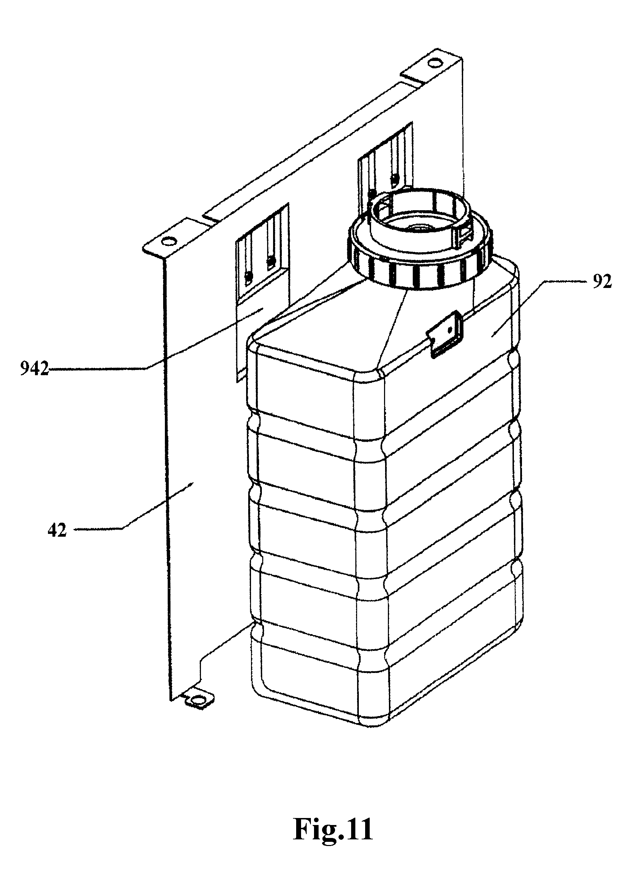

Referring to Fig. 10 and Fig. 11, there is an overflow detector 941 on the median clapboard 42 for detecting the liquid level in the sewage bucket 50. When the liquid level reaches a certain value, the controller will make a sound signaling to open the buckles 102 to close the valve 49. After taking out the sewage bucket 50 from the housing, the lid of the drain pipe 52 connected to the bottom of the sewage bucket 50 is released to drain the sewage in the pail. The overflow detector 941 is a non-contact type of detector. For the sake of security, two of such overflow detector 941 may be configured. Moreover, the overflow detector may be a capacitive approach switch 942, which also has the alarm function.

The outlet 551 of the three-way solenoid valve 55 is connected to one of the joints 500 (which is located above the joint 501)of the excretion pool 53 via a conduit (not shown), and the inlet 552 of the three-way solenoid valve 55 is connected with the air outlet of the vacuum pump 34. There is a negative ion generator 18 provided at the air suction port of the vacuum pump 34. A winder 12 and a winding sensor 13 are secured between the chassis 11 and the base 14 for winding up the power line, the chassis 11 being located at the bottom of the housing 44. The median clapboard 41, 42 divide the housing into three parts. The sink 31 and some parts are located at the left side of the median clapboard 41; the sewage bucket 50 and some parts are located at the right side of the median clapboard 42; and, the compressor 25, the vacuum pump 34, the electromagnetic pump 36 and the controller etc. are mounted between the two median clapboard 41 and 42. A fan 16 is mounted at the inside of the mesh 441 of the housing 44 for dissipating the heat generated by the compressor and other parts. A handle 61, an upper bracket 62, a connecting frame 63, a rod head 64, an upper slide bar 65, an upper steering knuckle 66, a lower steering knuckle 67, a lower slide bar 68, a fixing frame 69 and a slide bar sleeve 72 form a set of hand telescoping rod for moving the automatic human excrement disposal machine.

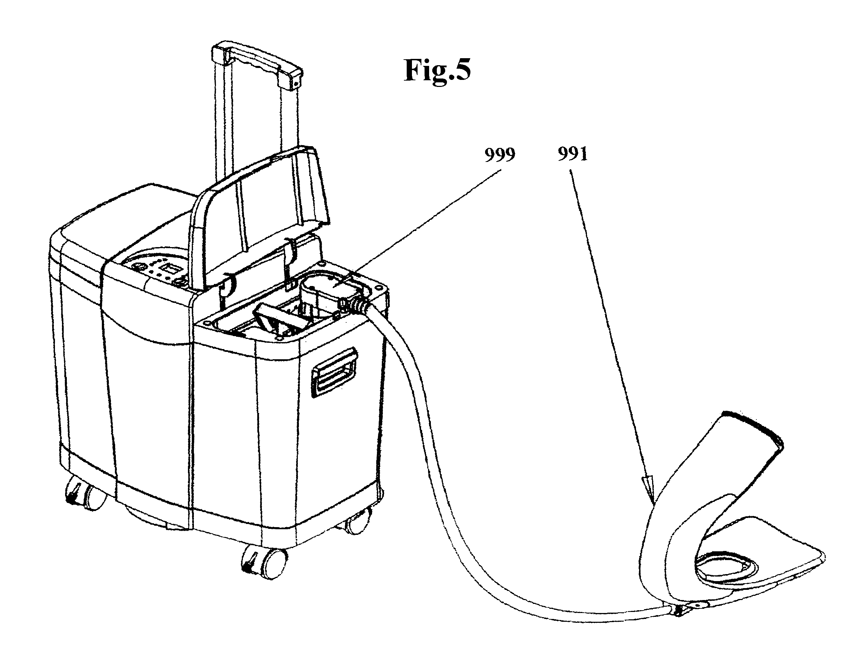

Fig.5 is a schematic view illustrating the overall profile of the automatic human excrement disposal machine in accordance with the present invention. The host machine 999 and the seat pad 991 are connected via a corrugated pipe and a high quality polyurethane (PU) tube, which are convenient to handle, disassembly and wash, clean and package.

Fig. 6A, 6B and 6C are schematic views illustrating the structure of the seat pad in accordance with present invention from different view angles. The seat pad 991 takes an approximately "U" shape, including a lower part 997 partially located under the user during usage and an upper part 996 that partially located above the user during usage, and also a excretion pool 91 with an approximate slope. The upper part and the lower part include passages (see passage 989 in Fig.3) located therein for passing water and air therethrough. The excretion pool 91 includes a sewage draining nozzle 995. The seat pad is made of polyurethane (PU) material by moulding. Of course, other materials and manufacture method known to those skilled in the art can also be applied. The lower part 997 includes two water/air jet nozzles 992 (namely two water/air passages) arranged in parallel and an air jet nozzle 993 (namely an air passage) located between the two nozzles. The configuration of the nozzles is such that when the nozzles get into operations, water and air are ejected into the passages at an angle a5 (relative to the downslope) about 0°to about 60° relative to the vertical plane, and at an angle a4 about 0° to about 90° relative to the horizontal plane. The upper part 996 includes a common passage (i.e., the water/air inlet nozzle 994) for both water and air. The nozzle 994 is so configured that water and air is ejected at an angle a6 (relative to the horizontal plain of the location of the nozzle)about -45° to about 45° relative to the vertical plane. The sewage draining nozzle 995 of the sewage draining passage has a diameter of about 0.5 cm to 6 cm and has a longitudinal axis forming an angle a3 of about 0.5°to about 89°relative to the horizontal plane. The internal surface of the upper part 996 and the horizontal plane form an angle a1 of about 20°to about 90°; the downslope of the excretion pool 91 and the horizontal plane form an angle a2 of about 0.2°to about 80°; the height h of the lower part 997 is about 1 cm to about 50 cm. It should be understood that the location, the shape and the dimension of the water jet nozzles, the air jet nozzles described above can vary according to specific situations.

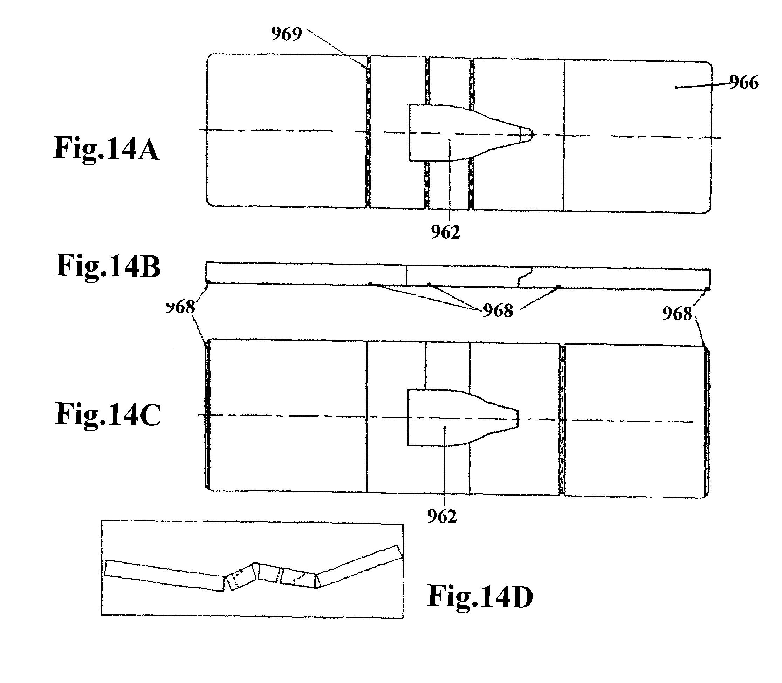

Fig. 14A to 14D are different views of the tournure in accordance with the present invention. The tournure 966 can be fold up along the connecting line 969. Fig. 14D is a schematic view illustrating the folding profile, with the joints clearly shown. The tournure is made of gray flocking fabric or flocking fabric of any other color, provided that the fabric must be waterproof, crease resistant, and anti-electrostatic. An opening 962 is formed at the center of the tournure for securing the seat pad 991. The zipper 968 can be a coarse teeth zipper that can be smoothly zipped up. The color of the zipper may be close to the fabric for the tournure. The zipper slider can be covered with elastic cloth. During the assembly of the tournure, the zipper 968 is unzipped to let sponge or some other soft materials be put inside. Six pieces of sponges are shown in the figures. Apparently, some changes can be made to the tournure based on the design described above.

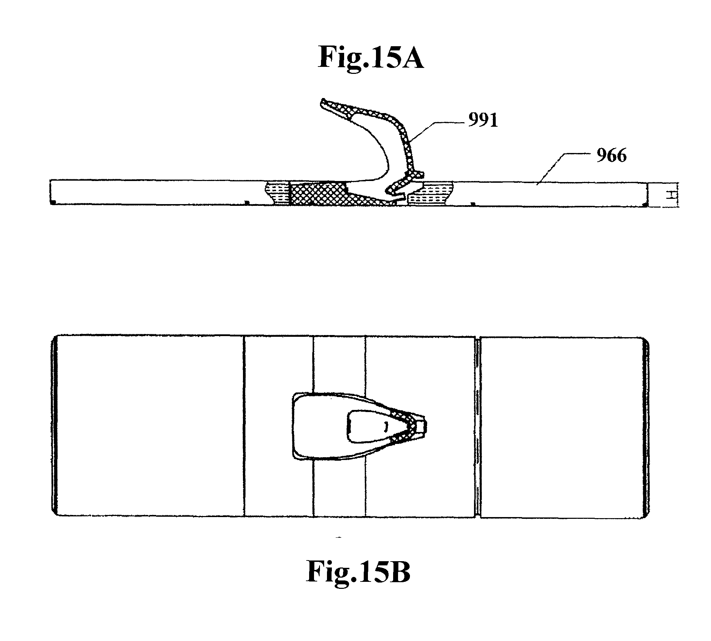

Fig.15A and 15B are different views of the combination of the tournure 966 and the seat pad 911 in accordance with the present invention, wherein, the height of the tournure is between about 1 cm and 50 cm.

Hereunder will be described another embodiment of the disposal machine for human excrement in accordance with present invention, with the emphasis of the description on the difference from the above embodiment. Referring to the structural principle diagram of Fig.3 and the structural exploded view of Fig.4, the disposal machine for human excrement includes: a seat pad 991, a sewage bucket 92,an air cleaner 94 and a compressor 93 (see Fig.4), which are mainly used to collect excrement from the seat pad; a water tank 21, a sink 95 located under the water tank and an electromagnetic pump 96 connected with the sink, which are mainly used to supply water to the seat pad 991; a vacuum pump 98 (see Fig. 4) and a heating component 90 (see Fig. 1), which are mainly used to feed air into the seat pad; and a control assembly, which makes sure of a coordinated operation of the above parts.

The air way of the disposal machine for human excrement is described as follows: in the standby state, the vacuum pump 98 drives the air in the seat pad 991 to flow through the sewage bucket 92, the air cleaner 94, and the heating component 90 where the air get heated, and finally back into the seat pad 991 to maintain warm and ventilated atmosphere within the seat pad. In the working state, the compressor 93 compels the air in the seat pad 991 to flow through the sewage bucket 92, the air cleaner 94, and finally back to the seat pad 991 in order to maintain a pressure balance in the seat pad. The disposal machine for human excrement also includes a heater 90 mounted in the sink 95. The machine gets into operation, water heated by the heater 90 is fed into the seat pad under the action of the electromagnetic pump 96, and then into the passages within the seat pad through the water pipe. The water pump 96 may be an electromagnetic pump. Furthermore, a thermoregulator (for example, an over temperature detector, see Fig.3)may be configured in the sink 95 to connect with the control assembly for regulating the water temperature. The above-mentioned heating component may a heat exchange air duct in the sink.

The water supply outlet of the electromagnetic pump 96 and the air outlet of the vacuum pump 98 are connected to a shared pipeline, which is connected with corresponding passages within the seat pad 991. In the standby state, air is fed into the seat pad 991 via the shared pipeline, while in the working state, water is supplied to the seat pad 991 via the shared pipeline. Furthermore, a one-way check valve 987 can be configured on the air pipeline between the air outlet of the vacuum pump 98 and the inlet of the shared pipeline in order to prevent the water from flowing backwards into the vacuum pump 98.

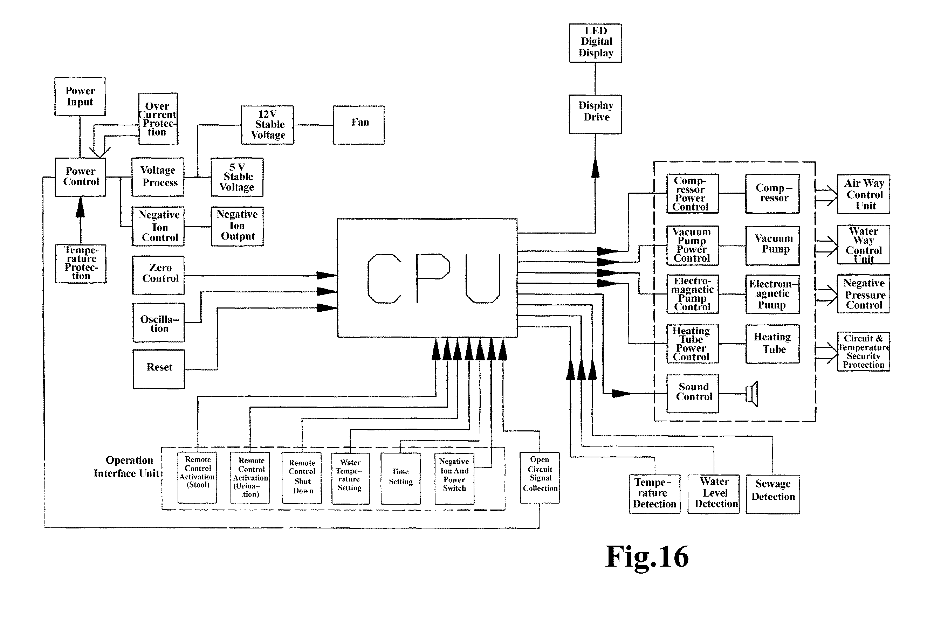

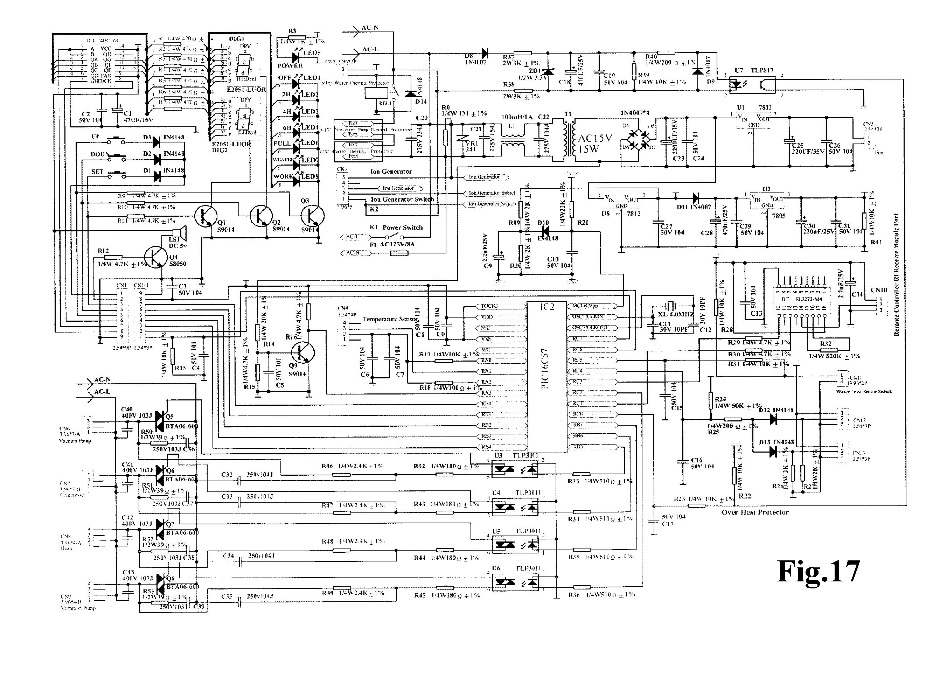

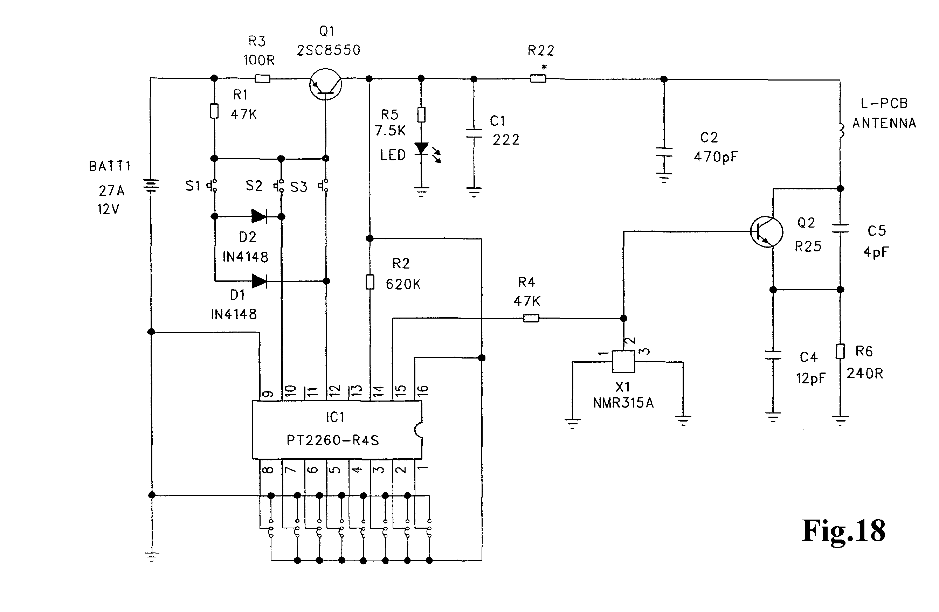

Fig. 16 to Fig. 18 illustrate a control module of the disposal machine for human excrement in accordance with the present invention. Fig.16 is a functional block diagram illustrating the control module of the disposal machine for human excrement in accordance with present invention. Fig. 17 is a schematic view illustrating the circuit principle layout of the control module of the disposal machine for human excrement in accordance with present invention. Fig.18 is a schematic diagram illustrating the circuit principle for the disposal machine for human excrement in accordance with present invention. As can be seen from the figures, the advantages of the control module in accordance with the present invention are as follows: in respect of the power supply, an isolating transformer is used, which is safe, stable and reliable; in respect of the circuit control, only one MCU is used to control the circuit, such that the circuit is simple, stable and reliable, and can control temperature and time accurately; in respect of the driving mode, it is achieved by using a MCU to control four controllable thyristors; in respect of circuit security, there are provided with an over-current protection circuit, an over-voltage protection circuit, an over-temperature protection circuit, a dry burning protection circuit and a circuit open alarm circuit and some other circuits in the processor of the present invention; in respect of air way control, only a one-way check valve is used, so the structure is very simple; in respect of water supply, only one electromagnetic pump is used, so the structure is fairly simple; in respect of key-press control, a wireless remote controller is used, which is very convenient to the user. Finally, the processor also employs negative ion technique, which is especially effective in freshing and sterilizing the air.

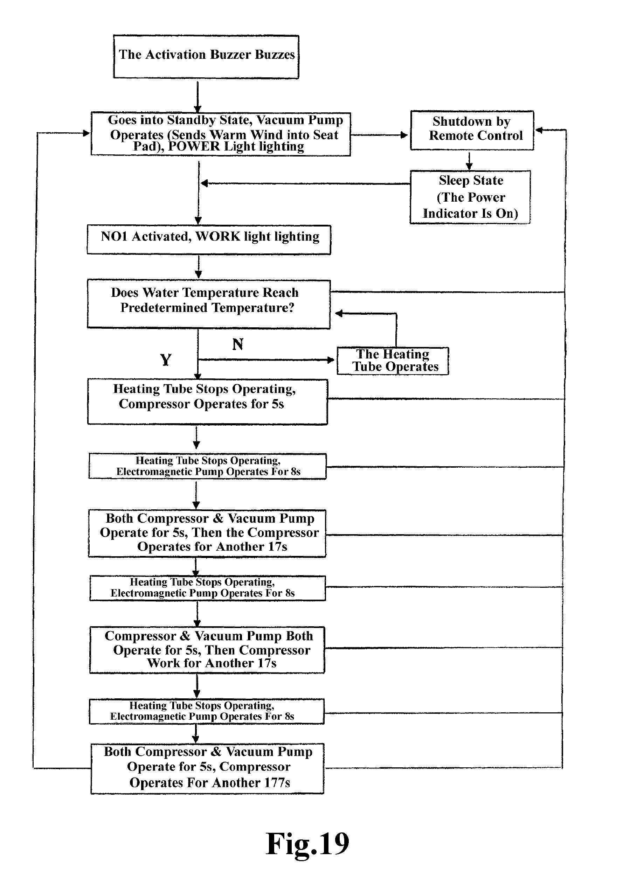

Fig.19 is a flow chart illustrating the normal work flow 1 of the disposal machine for human excrement in accordance with the present invention. A remote control NO1 (generally refers to the stool washing mode) starts the operation and ends the operation about 3min and 45s later. During operation, the vacuum pump starts to supply warm wind to the seat pad; the heating tube heats the water; the electromagnetic pump injects water into the seat pad; and the compressor generates negative pressure in the seat pad and feeds air into the seat pad simultaneously. It bears mentioning that the time periods such as 5s, 8s, or 17s, as exemplified in the embodiment, are illustrative only. Those skilled in the art can make changes thereto without departing from the protection scope of the present invention.

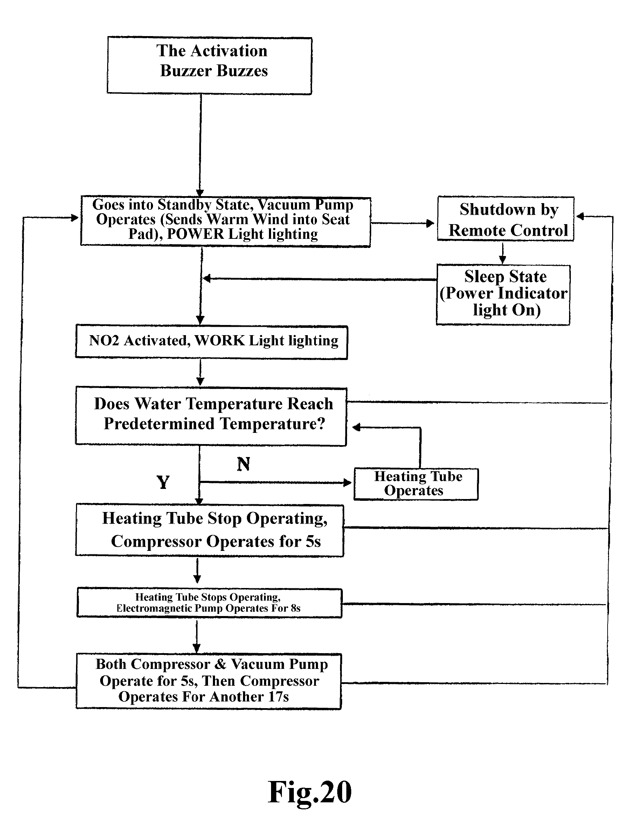

Fig.20 is a flow chart illustrating the normal work flow 2 of the disposal machine for human excrement in accordance with present invention. A remote control NO2 (generally refers to the urination washing mode) starts the operation and ends it about 35s later. During operation, the vacuum pump supplies warm wind to the seat pad; the heating tube heats water; the electromagnetic pump injects water into the seat pad; and the compressor generates negative pressure in the seat pad and also feeds air to the seat pad simultaneously.

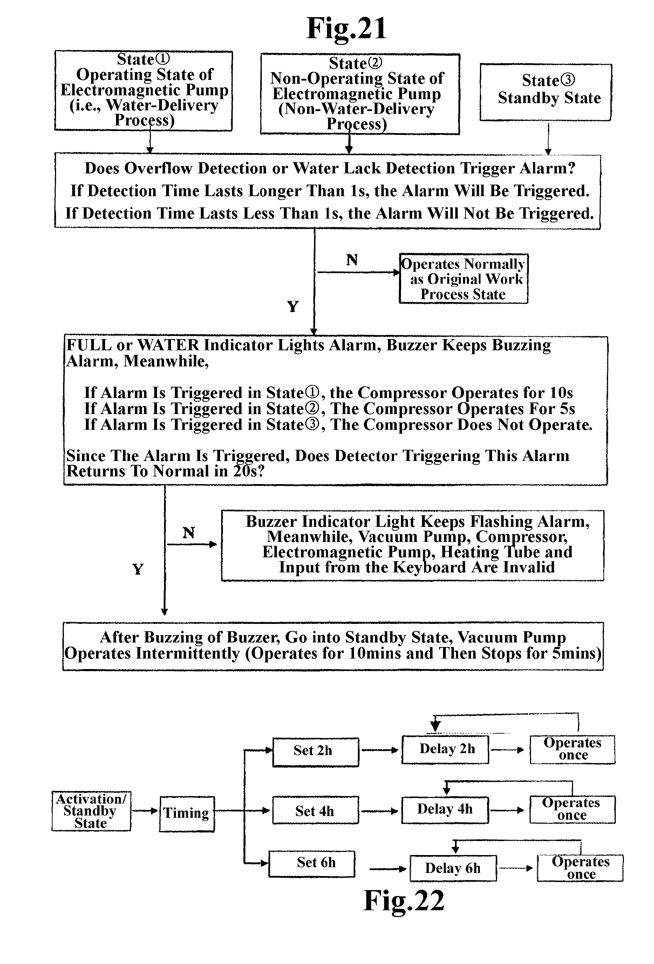

The alarm module of the disposal machine for human excrement in accordance with the present invention mainly includes an over temperature detection trigger alarm and an overflow and water lack alarm. Fig.21 is a view illustrating the workflow of the overflow or water lack alarm of the disposal machine for human excrement in accordance with the present invention. There are three states: state ① refers to a water-delivery state, state ② refers to a non-water-delivery state, and state ③ refers to a standby state. It should be noted that when the remote control is turned off, no alarm detection signal will be detected; when it is turned on, whether the detector works normally is first detected. If true, the regular operations are effected. Otherwise, the alarm program is started to stop the operations of any other loads.

The control flow of the over temperature detection trigger alarm is as follows: during the standby or working state, if an over temperature trigger signal is detected, alarming will be effected immediately. The buzzer keeps buzzing and all the indicator lights keep flashing to alarm. Only when the temperature returns to a normal range, and when the buzzer buzzes once, will the processor gets into the standby state. Thereafter, the vacuum pump will operate intermittently according to the MCU control mode.

It should be pointed out that, when alarm is detected, the buzzing, the flashing of indicator lights, and the operating time of the compressor are all varied in the above-mentioned three different states. At the very moment of activating the remote controller, if alarm is detected, the buzzer will buzz alarm, the corresponding indicator lights will flash, and the compressor will not operate. If no trigger alarm signal is detected within 20s, the circuit will return to the normal standby state, when the buzzer buzzes for once. Thereafter, the vacuum pump starts operating intermittently. The water temperature as detected must reach a predetermined temperature before running into normal operations. Otherwise, the heating tube will operate and will not stop until the predetermined temperature is reached. During operation, if the temperature is low, the heating tube will heat automatically (incubation). If the temperature sensor circuit opens, the digital dial indicators will keep flashing to alarm and the buzzer will keep buzzing to alarm (in such situation, the processor needs disassembling and repairing). During timing, if there is an overflow or water lack alarm, the timing must restart after the detector of the trigger alarm recovers. During trigger alarm, signals in multiple paths are capable of being detected simultaneously, and a corresponding LED can indicate the alarm accordingly. The states concerning the operations are defined as follows:

When the remote control is turned on, the processor is in the working state;

When the remote control is turned off, the processor is in the sleeping state, where the loads do not work;

When the task is finished, the processor is the standby state, where the vacuum pump operates intermittently according to a MCU control mode.

When the remote control is turned off or the power supply is turned on, all the previous programs are reset to restart operation/timing/temperature measuring.

Fig.22 is a schematic block diagram illustrating the timing (delaying) operation of the disposal machine for human excrement in accordance with the present invention. In case of a timed activation, if the preset time arrives but the temperature has not reached the predetermined temperature, the WORK indicator light will be turned on, and only when the temperature reaches the predetermined temperature, will a subsequent process be started (e.g., the operation of 3min and 45s). A new timing operation will be effected after the current task is finished. Timing for an operation may be executed at any time using the timing key. Once the indicator light starts flashing, the timing will restart according to the time currently set.

Although the present invention has been described in connection with several embodiments, it should not be understood that present invention is restricted to such embodiments. It should be point out that the specific time periods and data as exemplified in the embodiments are illustrative only. Those skilled in the art can make any changes thereto without departing from the protection scope of present invention. Therefore, the present invention covers all the alternatives, modifications and equivalents made within the spirit and scope of the appended claims.

## Claims

1. A disposal machine for human excrement, comprising: a excretion pool, a sewage bucket and a controller, wherein a blow-off pipe is such configured that it has one end connected to the bottom of the excretion pool within the seat pad and the other end connected to the sewage bucket, and wherein the top of the sewage bucket is connected with a compressor via a fitting pipe, the compressor connected to an air cleaner.

2. The disposal machine for human excrement according to claim 1, wherein: the blow-off pipe is connected to the top of the sewage bucket.

3. The disposal machine for human excrement according to claim 1, wherein:
a water pipe is such configured that it has one end connected to the upper part of the excretion pool and the other end to an outlet of an electromagnetic pump with its water inlet connected with a sink.

4. The disposal machine for human excrement according to claim 3, wherein: a heater is provided in the sink.

5. The disposal machine for human excrement according to claim 3, wherein: an air pipe is configured such that it has one end connected to the excretion pool and the other end to a vacuum pump.

6. The disposal machine for human excrement according to claim 5, wherein: a heater is defined in an air inlet passage or an air outlet passage of the vacuum pump.

7. The disposal machine for human excrement according to claim 1, wherein:
a conduit is configured such that it has one end connected to the upper part of the excretion pool and the other end to an outlet of a three-way valve, two inlets of the three-way valve connected with the water outlet of the electromagnetic pump and the air outlet of the vacuum pump respectively via a water pipe and an air pipe respectively, and the water inlet of the electromagnetic pump is connected with the sink.

8. The disposal machine for human excrement according to claim 5 or 7, wherein: a heater is provided in the sink; and part of the air pipe is immersed in the water of the sink.

9. The disposal machine for human excrement according to claim 5 or 7, wherein: a negative ion generator is provided in the inlet passage or the outlet passage of the vacuum pump.

10. A disposal machine for human excrement, comprising:
a seat pad;
a sewage disposal assembly to collect excrement from the seat pad;
a water supply assembly to feed water into the seat pad;
an air supply assembly to feed air into the seat pad; and
a control assembly to control operations of the sewage disposal assembly, water supply assembly and air supply assembly.

11. The disposal machine for human excrement according to claim 10, wherein: the sewage disposal assembly includes a sewage bucket, an air cleaner and a compressor; the water supply assembly includes a water tank, a sink located under the water tank and an electromagnetic pump connected with the sink; and the air supply assembly includes a vacuum pump and a heating component.

12. The disposal machine for human excrement according to claim 11, wherein:
in a standby state, the vacuum pump drives the air in the seat pad to flow through the sewage bucket, the air cleaner, and then through the heating component where the air gets heated, and finally flow back into the seat pad in order to maintain a warm and ventilated atmosphere in the seat pad; and in a working state, the compressor drives the air in the seat pad to flow through the sewage bucket, the air cleaner, and finally back into the seat pad in order to main a pressure balance in the seat pad; and wherein:
the water supply assembly further includes a heater defined in the sink for heating water that, once heated, is driven by the electromagnetic pump to flow into the seat pad via the water pipe and the passages within the seat pad in the working state.

13. The disposal machine for human excrement according to claim 12, wherein:
the water supply outlet of the water supply assembly and the air supply outlet of the air supply assembly are both connected to a shared pipeline, the outlet of which is communicated with corresponding passages within the seat pad, and an one-way check valve is defined on the air pipeline between the outlet of the air supply assembly and the inlet of said shared pipeline, and wherein
in the standby state, the air supply assembly feeds air into the seat pad via the shared pipeline, while in the working state, the water supply assembly feeds water into the seat pad via the shared pipeline.

14. The disposal machine for human excrement according to any of claims 11 to 13, wherein:
the seat pad is approximately U- or V-shaped, and includes a lower part partially located under the user during use, an upper part partially located above the user during use, and a excretion pool substantially taking the form of a slope, the upper part and lower part including passages located therein for passing water and air therethrough, the excretion pool including a sewage draining passage.

15. The disposal machine for human excrement according to claim 14, wherein the seat pad is made of polyurethane (PU).

16. The disposal machine for human excrement according to claim 14, wherein: the lower part is provided with three parallel water and/or air passages, the passages being such configured that when in use water/air is ejected from the passages at an angle ranging from about 0°to about 60° relative to the vertical plane, and an angle ranging from about 0°to about 90° relative to the horizontal plane; and the upper part includes a shared passage for both water and gas, about the passage being such configured that water/air is ejected from the passage at an angle ranging from about -45°to about 45°with respect to the horizontal plane, and wherein:
the sewage draining nozzle of the sewage draining passage has a diameter of about 0.5 cm to about 6 cm, and has a central axis forming an angle of about0.5°to about 89°relative to the horizontal plane; the internal surface of the upper part forms an angle of about 20°to about 90°relative to the horizontal plane; the downslope of the excretion pool forms an angle about 0.2°to about 80° relative to the horizontal plane, and the height of the lower part is about 1 cm to about 50 cm.

17. The disposal machine for human excrement according to any one of the claims from 11 to 13, wherein the sewage disposal assembly also includes an overflow detector for detecting the liquid level in the sewage bucket.

18. The disposal machine for human excrement according to any one the claims from 11 to 13, wherein: a thermoregulator is defined in the sink for regulating the water temperature.

19. The disposal machine for human excrement according to any one of the claims from 11 to 13, wherein a water level detector is defined in the water supply assembly for controlling water level.

20. The disposal machine for human excrement according to any one of the claims from 11 to 13, wherein a bent pipe inserted from the top of the sewage bucket has a diameter between about 1 mm and about 80 mm, and is located at a distance between about 1 cm and about 50 cm from the bottom of said sewage bucket.

21. The disposal machine for human excrement according to any one of the claims from 11 to 13, wherein: the diameter of the opening in the water tank lid of the water tank is between about 1 mm and about 80 mm.

22. The disposal machine for human excrement according to any one of the claims from 11 to 13, wherein the control assembly includes a remote controller for controlling the disposal machine for human excrement.

23. The disposal machine for human excrement according to any one of the claims from 11 to 13, further comprising a negative ion generator for purifying surrounding environment.

24. A tournure for the disposal machine for human excrement according to any one of the claims from 11 to 13, which has an appropriate opening for accommodating the seat pad or an integral combination of the above seat pad and the tournure.

25. A method for operating a disposal machine for human excrement, which includes working state when activated, sleep state when shut down and standby state when a task is finished.

26. The method according to the claim 25, wherein:
in the working state, a suction device operates to generate a negative pressure in the seat pad and drives air into the seat pad, when the electromagnetic pump operates to feed water into the seat pad simultaneously; and in the standby state, an air supply device operates to supply warm wind into the seat pad.

27. The method according to claim 26, comprising:
detecting whether the sewage bucket is full of water using an overflow detector;
detecting whether the sink is lack of water using a water level detector;
detecting whether the water temperature is too high using an over temperature detector.

28. The method according to any one of the claims from 25 to 27, further comprising: timing and delaying operations using a delay control module.
